# EUROPÄISCHE PATENTANMELDUNG

(11) **EP 2 808 045 A1**
(43) Veröffentlichungstag der Anmeldung: **03.12.2014**
(21) Anmeldenummer: 14168400.1
(22) Anmeldetag: 15.05.2014
(51) Int. Cl.: A61M 5/20, A61M 5/32

(54) **Aufsatz für eine Spritze**

(30) Priorität: 27.05.2013 DE 102013008876
(71) Anmelder: Heußler, Tilmann, 77833 Ottersweier (DE)
(72) Erfinder: Heußler, Tilmann, 77833 Ottersweier (DE)
(74) Vertreter: Reule, Hanspeter

(57) **Zusammenfassung**

Die Erfindung betrifft einen Aufsatz (20) für eine einen Spritzenkörper (12) für die Aufnahme eines Medikaments mit einer Auslassöffnung an seinem vorderen Ende (18) und eine an der Auslassöffnung befestigte Nadel (16) aufweisende Spritze (10), mit einem Gehäuse (24) zur Aufnahme des vorderen Endes (18) des Spritzenkörpers (12), das einen Mantel (26) zum Umhüllen des Spritzenkörpers (12) und eine Stirnseite (28) mit einer Durchtrittsöffnung (32) für den Durchtritt der Nadel (16) aufweist, mit einer im Gehäuse (24) in axialer Richtung von der Stirnseite (28) weg und zur Stirnseite (28) hin linear verschieblich angeordneten Halteeinrichtung (34) zum Fixieren des Spritzenkörpers (12) und mit einer mindestens eine Rückstellfeder (40) aufweisenden Federanordnung, die bei Bewegung der Halteeinrichtung (34) von der Stirnseite (28) weg gespannt wird und auf die Halteeinrichtung (34) eine zur Stirnseite (28) gerichtete Rückstellkraft ausübt, wobei die mindestens eine Rückstellfeder (40) eine Druckfeder ist und zwischen der Halteeinrichtung (34) und dem Gehäuse (24) abgestützt ist. Erfindungsgemäß ist vorgesehen, dass das Gehäuse (24) eine in axialer Richtung unverschiebliche Führungseinrichtung für den Spritzenkörper (12) aufweist, die als gehäusefester Führungsring (38) ausgebildet ist, und dass die mindestens eine Rückstellfeder (40) zwischen der Halteeinrichtung (34) und dem Führungsring (38) abgestützt ist.

## Beschreibung

Die Erfindung betrifft einen Aufsatz für eine Spritze gemäß Oberbegriff des Anspruchs 1.

Es besteht vielfach die Notwendigkeit, dass sich Patienten selbst ein Medikament spritzen. Dies ist beispielsweise bei Diabetespatienten der Fall, die sich regelmäßig Insulin spritzen müssen. Aber auch Spritzen zur Vorsorge gegen Thrombose werden oft vom Patienten selbst angewendet. Dabei kommen meist gewöhnliche Spritzen zur Anwendung, wie sie auch durch medizinisches Fachpersonal verwendet werden. Diese Spritzen, die einen meist zylinderförmigen Spritzenkörper für die Aufnahme des Medikaments mit einer Auslassöffnung bzw. Düse an dessen vorderem Ende sowie eine an der Auslassöffnung befestigte Nadel bzw. Kanüle aufweisen, sind auch von Laien einfach zu handhaben. Es muss lediglich die Nadel durch die Haut gestochen werden und das Medikament durch Vorschub eines im Spritzenkörper linear beweglichen Kolbens über die Auslassöffnung und die Nadel unter die Haut injiziert werden. Für einige Patienten stellt aber gerade das Einstechen der Nadel in die Haut eine solch unangenehme Handlung dar, dass sie davor zurückschrecken, selbst eine Injektion vorzunehmen.

Aus der DE 20 2009 001 836 U1 ist ein Aufsatz für eine Spritze der eingangs genannten Art bekannt, mit dem das Einstechen der Nadel mechanisiert werden kann. Der Aufsatz weist ein Gehäuse auf, in dem eine Spritze aufgenommen ist. Die Spritze ruht auf einem Einstechschlitten, der mit einem Injektionsschlitten zusammenwirkt, wobei Letzterer durch eine Druckfeder in Richtung zum vorderen Ende des Gehäuses mit Kraft beaufschlagt wird und bei einer durch die Druckfeder angetriebenen Vorwärtsbewegung den Einstechschlitten mitnimmt, so dass die Nadel der Spritze aus dem Gehäuse austritt. Der Aufbau dieses Aufsatzes ist somit kompliziert, so dass der Aufsatz teuer zu fertigen ist.

Aus der WO 2005/058 396 A1 ist ein auslösbares Injektionsgerät bekannt, dessen Nadel aus der Stirnseite eines Gehäuses gegen eine durch eine Rückstellfeder aufgebrachte Rückstellkraft herausbeweglich ist.

Es ist daher Aufgabe der Erfindung, einen Aufsatz der eingangs genannten Art derart weiterzubilden, dass er einfacher zu fertigen ist.

Diese Aufgabe wird erfindungsgemäß durch einen Aufsatz mit den Merkmalen des Anspruchs 1 gelöst. Vorteilhafte Weiterbildungen der Erfindung sind Gegenstand der abhängigen Ansprüche.

Der Erfindung liegt der Gedanke zugrunde, den Vorgang des Einstechens der Nadel bzw. Kanüle in die Haut zu mechanisieren, so dass er nicht direkt vom Anwender selbst durchgeführt werden muss, sondern nach Auslösung durch den Anwender selbsttätig erfolgt. Zu diesem Zweck wird ein Aufsatz auf den Spritzenkörper aufgesetzt, der das vordere, die Nadel tragende Ende des Spritzenkörpers aufnimmt. Der Aufsatz weist eine Halteeinrichtung auf, an der der Spritzenkörper fixiert wird, und die in einem den Spritzenkörper zumindest teilweise umschließenden Gehäuse linear verschieblich gelagert ist. Das Gehäuse weist an einer Stirnseite eine Durchtrittsöffnung für den Durchtritt der Nadel auf und die Halteeinrichtung wird durch die Kraft mindestens einer Rückstellfeder derart beaufschlagt, dass die Rückstellfeder beim Entfernen der Haltevorrichtung von der Stirnseite gespannt wird. Durch die Rückstellkraft wird die Halteeinrichtung mit der an ihr fixierten Spritze zur Stirnseite hin bewegt, so dass die Nadel aus der Durchtrittsöffnung austreten und in die Haut des Anwenders eindringen kann, wenn die Stirnseite auf die Haut aufgesetzt ist.

Die mindestens eine Rückstellfeder ist eine Druckfeder, welche zwischen der Halteeinrichtung und dem Gehäuse abgestützt ist. Ein Verschieben der Halteeinrichtung von der Stirnseite weg führt zu einer Kompression der Druckfeder und dem Aufbau der Rückstellkraft. Des Weiteren weist das Gehäuse eine in axialer Richtung unverschiebliche Führungseinrichtung für den Spritzenkörper auf. Die Führungseinrichtung ist als gehäusefester Führungsring ausgebildet, welcher in größerem Abstand zur Stirnseite angeordnet ist als die Halteeinrichtung. Die Führungseinrichtung stellt eine Linearführung für den Spritzenkörper dar, so dass dessen Vorschubbewegung besser definiert ist. Dabei ist die mindestens eine Rückstellfeder zwischen der Halteeinrichtung und dem Führungsring abgestützt, so dass der Führungsring zwei Funktionen in sich vereint.

Vorzugsweise weist der Aufsatz einen Haltemechanismus zum gehäusefesten Fixieren der Halteeinrichtung bei gespannter Rückstellfeder bzw. bei gespannten Rückstellfedern auf. Mittels des Haltemechanismus ist es möglich, zwischen dem Zurückziehen der Halteeinrichtung und damit der Spritze und dem durch das Vorschieben der Halteeinrichtung bewirkten Stechprozess einen zeitlichen Abstand herzustellen bzw. das Zurückziehen durch eine andere Person vornehmen zu lassen als den Stechprozess. Damit kann dem Anwender ein bereits vorgespannter Aufsatz mit in ihm aufgenommener Spritze übergeben werden, bei dem die Nadel vollständig im Gehäuse aufgenommen ist, so dass er den Stechvorgang auf einfache Weise durch Auslösen des Haltemechanismus, also durch einen in seinen Gedanken nicht unmittelbar mit dem Einstechen der Nadel in die Haut verbundenen Vorgang auslösen kann. Dabei weist der Haltemechanismus zweckmäßig mindestens einen radial von der Halteeinrichtung abstehenden, in einer Führung im Gehäuse geführten Zapfen auf. Zudem wird bevorzugt, dass jede Führung einen ersten, sich in axialer Richtung erstreckenden Abschnitt und einen zweiten, sich quer zur axialen Richtung erstreckenden Abschnitt aufweist und dass die Halteeinrichtung drehbar im Gehäuse angeordnet ist. Dies stellt eine besonders einfache und leicht zu handhabende Ausgestaltung dar.

Gemäß einer vorteilhaften Weiterbildung weist das Gehäuse zudem einen den Vorschub der Halteeinrichtung zur Stirnseite begrenzenden Anschlagring auf. Damit stößt die Halteeinrichtung bzw. die Spritze beim Vorschieben nicht an der Stirnseite des Gehäuses an.

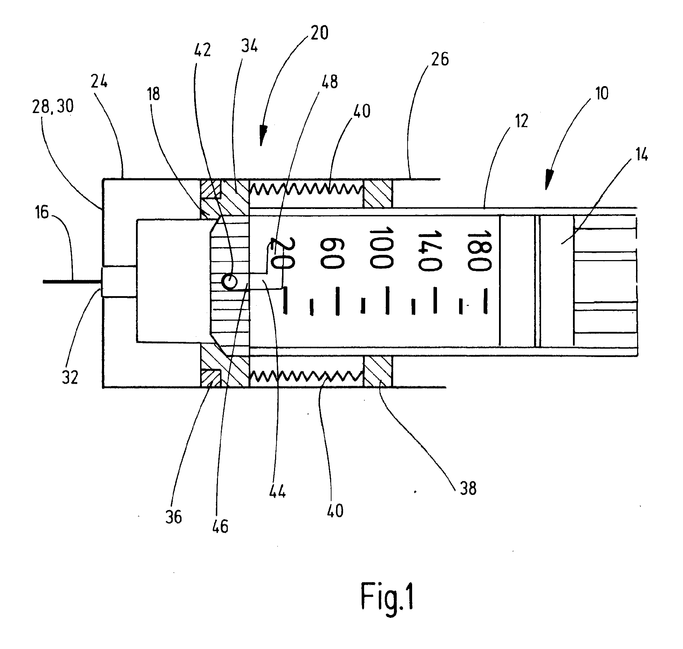

Im Folgenden wird die Erfindung anhand eines in der Zeichnung schematisch dargestellten Ausführungsbeispiels näher erläutert. Es zeigt
- Fig. 1: eine in einem Aufsatz aufgenommene Spritze, wobei der Aufsatz teilweise im Schnitt dargestellt ist.

In Fig. 1 ist eine Spritze 10 dargestellt, die einen Spritzenkörper 12 mit zylindrischem, für die Aufnahme eines flüssigen Medikaments bestimmten Innenraum, einen im Spritzenkörper 12 verschiebbar aufgenommenen Kolben 14 und eine Nadel 16 aufweist. Der Spritzenkörper 12 weist an seinem vorderen Ende 18 eine Auslassöffnung für das Medikament auf, welche in die Nadel 16 mündet.

Der Spritzenkörper 12 ist mit seinem vorderen Ende 18 in einem Aufsatz 20 aufgenommen, der ein Gehäuse 24 aufweist, das mit einem Mantel 26 den Spritzenkörper 12 rings umschließt. An einer Stirnseite 28 weist das Gehäuse 24 eine an den Mantel 26 angrenzende Stirnfläche 30 auf, welche mittig mit einer Durchtrittsöffnung 32 versehen ist, durch die in der Darstellung gemäß Fig. 1 die Nadel 16 ragt. Im Gehäuse 24 ist eine Halteeinrichtung 34 in Form eines Rings längsverschieblich angeordnet, in welcher der Spritzenkörper 12 fixiert ist. Der Vorschub der Halteeinrichtung 34 in Richtung zur Stirnseite 28 wird durch einen Anschlagring 36 begrenzt, welcher fest mit dem Mantel 26 verbunden ist. Zudem ist der Spritzenkörper 12 in einem Führungsring 38 geführt, welcher ebenfalls fest mit dem Mantel 26 verbunden ist und in größerem Abstand zur Stirnseite 28 angeordnet ist als die Halteeinrichtung 34. Zwischen der Halteeinrichtung 34 und dem Führungsring 38 sind Rückstellfedern 40 angeordnet, die als Druckfedern ausgebildet sind und sich auf der Halteeinrichtung 34 und dem Führungsring 38 abstützen. Gegen die Rückstellkraft der Rückstellfedern 40 kann die Halteeinrichtung 34 und mit ihr die an ihr fixierte Spritze 10 so weit von der Stirnseite 28 weg bewegt werden, bis die Nadel 16 vollständig im Gehäuse 24 aufgenommen ist.

Von der Halteeinrichtung 34 steht radial ein Zapfen 42 ab, welcher in eine Führung 44 im Gehäuse 24 eingreift und in ihr geführt ist. Die Führung 44 wird durch ein in Draufsicht L-förmiges Loch im Mantel 26 gebildet, wobei ein erster Abschnitt 46 sich in axialer Richtung und ein zweiter Abschnitt 48 sich senkrecht zum ersten Abschnitt 46 in tangentialer Richtung des Gehäuses 24 erstreckt. Der Zapfen 42 und die Führung 44 bilden einen Haltemechanismus. Zum Spannen der in Fig. 1 gezeigten Vorrichtung wird die Halteeinrichtung 34 unter Spannen der Rückstellfedern 40 von der Stirnseite 28 weg bewegt, wobei der Zapfen 42 entlang des ersten Abschnitts 46 der Führungskulisse 44 bewegt wird. Ist die Nadel 16 im Gehäuse 24 aufgenommen, so wird der Zapfen 42 durch Verdrehen der Halteeinrichtung 34 gegenüber dem Gehäuse 24 in den zweiten Abschnitt 48 der Führungskulisse 44 bewegt, so dass die Halteeinrichtung 34 in der zurückgezogenen Position fixiert ist. Durch ein Zurückdrehen wird der Haltemechanismus entriegelt und die Rückstellkraft der Rückstellfedern 40 bewirkt einen Vorschub der Halteeinrichtung 34 und der an ihr fixierten Spritze 10, bis die in Fig. 1 dargestellte vorgeschobene Position erreicht ist. Wird die Stirnfläche 30 auf die Haut eines Patienten aufgesetzt, so sticht die Nadel 16 bei der Vorschubbewegung in die Haut.

Zusammenfassend ist folgendes festzuhalten: Die Erfindung betrifft einen Aufsatz 20 für eine einen Spritzenkörper 12 für die Aufnahme eines Medikaments mit einer Auslassöffnung an seinem vorderen Ende 18 und mit einer an der Auslassöffnung befestigten Nadel 16 aufweisende Spritze 10, mit einem Gehäuse 24 zur Aufnahme des vorderen Endes 18 des Spritzenkörpers 12, das einen Mantel 26 zum Umhüllen des Spritzenkörpers 12 und eine Stirnseite 28 mit einer Durchtrittsöffnung 32 für den Durchtritt der Nadel 16 aufweist, mit einer im Gehäuse 24 in axialer Richtung von der Stirnseite 28 weg und zur Stirnseite 28 hin linear verschieblich angeordneten Halteeinrichtung 34 zum Fixieren des Spritzenkörpers 12 und mit einer mindestens eine Rückstellfeder 40 aufweisenden Federanordnung, die bei Bewegung der Halteeinrichtung 34 von der Stirnseite 28 weg gespannt wird und auf die Halteeinrichtung 34 eine zur Stirnseite 28 gerichtete Rückstellkraft ausübt.

### Bezugszeichenliste

- 10: Spritze
- 12: Spritzenkörper
- 14: Kolben
- 16: Nadel
- 18: vorderes Ende
- 20: Aufsatz
- 24: Gehäuse
- 26: Mantel
- 28: Stirnseite
- 30: Stirnfläche
- 32: Durchtrittsöffnung
- 34: Halteeinrichtung
- 36: Anschlagring
- 38: Führungsring
- 40: Rückstellfeder
- 42: Zapfen
- 44: Führung
- 46: erster Abschnitt
- 48: zweiter Abschnitt

## Patentansprüche

1. Aufsatz für eine einen Spritzenkörper (12) für die Aufnahme eines Medikaments mit einer Auslassöffnung an seinem vorderen Ende (18) und eine an der Auslassöffnung befestigte Nadel (16) aufweisende Spritze (10), mit einem Gehäuse (24) zur Aufnahme des vorderen Endes (18) des Spritzenkörpers (12), das einen Mantel (26) zum Umhüllen des Spritzenkörpers (12) und eine Stirnseite (28) mit einer Durchtrittsöffnung (32) für den Durchtritt der Nadel (16) aufweist, mit einer im Gehäuse (24) in axialer Richtung von der Stirnseite (28) weg und zur Stirnseite (28) hin linear verschieblich angeordneten Halteeinrichtung (34) zum Fixieren des Spritzenkörpers (12) und mit einer mindestens eine Rückstellfeder (40) aufweisenden Federanordnung, die bei Bewegung der Halteeinrichtung (34) von der Stirnseite (28) weg gespannt wird und auf die Halteeinrichtung (34) eine zur Stirnseite (28) gerichtete Rückstellkraft ausübt, wobei die mindestens eine Rückstellfeder (40) eine Druckfeder ist und zwischen der Halteeinrichtung (34) und dem Gehäuse (24) abgestützt ist, **dadurch gekennzeichnet, dass** das Gehäuse (24) eine in axialer Richtung unverschiebliche Führungseinrichtung für den Spritzenkörper (12) aufweist, die als gehäusefester Führungsring (38) ausgebildet ist, und dass die mindestens eine Rückstellfeder (40) zwischen der Halteeinrichtung (34) und dem Führungsring (38) abgestützt ist.

2. Aufsatz nach Anspruch 1, **gekennzeichnet durch** einen Haltemechanismus zum gehäusefesten Fixieren der Halteeinrichtung (34) bei gespannter Rückstellfeder (40) bzw. bei gespannten Rückstellfedern (40).

3. Aufsatz nach Anspruch 2, **dadurch gekennzeichnet, dass** der Haltemechanismus mindestens einen radial von der Halteeinrichtung (34) abstehenden, in einer Führung (44) im Gehäuse (24) geführten Zapfen (42) aufweist.

4. Aufsatz nach Anspruch 3, **dadurch gekennzeichnet, dass** jede Führung (44) einen ersten, sich in axialer Richtung erstreckenden Abschnitt (46) und einen zweiten, sich quer zur axialen Richtung erstreckenden Abschnitt (48) aufweist und dass die Halteeinrichtung (34) drehbar im Gehäuse (24) angeordnet ist.

5. Aufsatz nach einem der vorangehenden Ansprüche, **dadurch gekennzeichnet, dass** das Gehäuse (24) einen den Vorschub der Halteeinrichtung (34) zur Stirnseite (28) begrenzenden Anschlagring (36) aufweist.

6. Spritzenanordnung mit einer einen Spritzenkörper (12) für die Aufnahme eines Medikaments mit einer Auslassöffnung an seinem vorderen Ende (18) und mit einer an der Auslassöffnung befestigten Nadel (16) aufweisenden Spritze (10) und mit einem Aufsatz (20) nach einem der vorangehenden Ansprüche, wobei der Spritzenkörper (12) an der Halteeinrichtung (34) fixiert ist und zwischen einer ersten Position, in der die Nadel (16) vollständig im Gehäuse (24) aufgenommen ist und einer zweiten Position, in der die Nadel (16) durch die Durchtrittsöffnung (32) hindurch ragt und ihr freies Ende außerhalb des Gehäuses (24) angeordnet ist, linear beweglich ist.
